# EUROPÄISCHE PATENTANMELDUNG

(11) **EP 2 998 018 A1**
(43) Veröffentlichungstag der Anmeldung: **23.03.2016**
(21) Anmeldenummer: 14185217.8
(22) Anmeldetag: 17.09.2014
(51) Int. Cl.: B01J 8/06, C07C 5/00

(54) **Verfahren und Anlage zur Alkandehydrierung**

(71) Anmelder: Linde Aktiengesellschaft, 80331 München (DE)
(72) Erfinder: Boelt, Heinz, 82515 Wolfratshausen (DE); Conradt, Joerg, 80639 München (DE); Hoefel, Torben, 81543 München (DE); Holzapfel, Ina, 81479 München (DE); Nold, Michael, 82515 Wolfratshausen (DE); Reyneke, Hendrik, 81479 München (DE); Stahl, Bernhard, 81379 München (DE); Wellenhofer, Anton, 82069 Hohenschäftlarn (DE); Hesse, Andreas, 82515 Wolfratshausen (DE)
(74) Vertreter: m patent group

(57) **Zusammenfassung**

Es wird ein Verfahren zur Alkandehydrierung, bei dem ein Einsatzstrom (1), der ein oder mehrere zu dehydrierende Alkane enthält, in zumindest einem Reaktor (10) durch eine Anzahl von Reaktionsrohren (11) geführt wird, die in einer Brennkammer (12) angeordnet sind und in dieser mit einer Anzahl von Brennern (15) beheizt werden, denen zumindest ein Brenngas (3) zugeführt wird, vorgeschlagen. Es ist vorgesehen, dass die Brenner (15) zumindest zeitweise flammenlos betrieben werden, indem einer Verbrennungszone der Brenner (15) ein Gasgemisch auf einer Temperatur, die oberhalb einer Zündtemperatur des Brenngases (3) liegt, zugeführt wird. Eine entsprechende Anlage (100) ist ebenfalls Gegenstand der Erfindung.

## Beschreibung

Die Erfindung betrifft ein Verfahren und eine Vorrichtung zur Alkandehydrierung gemäß den Oberbegriffen der unabhängigen Patentansprüche.

### Stand der Technik

Verfahren zur Dehydrierung von Alkanen, insbesondere von Propan zu Propylen und von Isobutan zu Isobuten, sind bekannt und beispielsweise im Artikel "Propene" in Ullmann's Encyclopedia of Industrial Chemistry, Onlineausgabe, 15. Juni 2000, DOI 10.1002/14356007.a22_211, und dort insbesondere in Abschnitt 4.3., "Propane Deydrogenation", beschrieben.

Die Dehydrierung von Alkanen wird typischerweise in Reaktoren durchgeführt, die mittels Brennern beheizte Brennkammern aufweisen, durch welche mehrere Reaktionsrohre geführt sind. Die Reaktionsrohre werden von außen durch die Brenner beheizt. Die Reaktionsrohre enthalten einen zur Alkandehydrierung geeigneten Katalysator auf einem entsprechenden Träger. Anforderungen an entsprechende Katalysatoren sind beispielsweise bei Bölt, H., "Dehydrierung von Propan/Butan", Linde Berichte aus Technik und Wissenschaft 66/1991, beschrieben. Durch die Reaktionsrohre wird das zu dehydrierende Alkan bzw. ein entsprechendes Gemisch aus mehreren Alkanen, beispielsweise Propan und/oder Butan, geführt. Zuvor erfolgt typischerweise eine Vorwärmung mittels Abwärme. Ein dem Reaktor bzw. entsprechenden Reaktionsrohren entnommenes Gasgemisch, nachfolgend auch als Produktstrom bezeichnet, wird einer geeigneten Produktaufbereitung zugeführt. Die Dehydrierung von Alkanen ist bei der Wahl geeigneter Katalysatoren und Reaktionsbedingungen hochselektiv, beispielsweise liegt die Ausbeute einer Reaktion von Propan zu Propylen typischerweise bei über 90%.

Selbst bei einer Vielzahl gezielt über die Brennkammer eines Reaktors zur Alkandehydrierung verteilter Brenner ist die Wärmeverteilung und Übertragung in der Brennkammer auf die einzelnen Reaktionsrohre jedoch häufig nicht gleichmäßig. Dies bedeutet, dass einzelne Reaktionsrohre einen deutlich höheren oder geringeren Wärmeeintrag als der Durchschnitt aller Reaktionsrohre erfahren. Insbesondere überdurchschnittlich stark erwärmte Reaktionsrohre weisen aufgrund der höheren Rohrwandtemperatur eine verringerte Lebensdauer auf, weil diese durch die höchste lokale Oberflächentemperatur des Materials festgelegt ist.

Ferner ergibt sich in den betroffenen Reaktionsrohren eine verringerte Lebensdauer des Katalysators. Aufgrund eines lokal erhöhten Wärmeeintrags kann es auch zu beschleunigter Verkokung kommen, weshalb die Produktionszeiten entsprechender Reaktoren verringert sind bzw. diese häufiger einer Regeneration unterworfen werden müssen. Gegenüber einer als ideal angenommenen Brennkammer mit vollständig gleichmäßiger Wärmeverteilung sind schließlich auch die erzielten Produktausbeuten in realen Reaktoren aufgrund entsprechender Ungleichmäßigkeiten verringert.

In herkömmlichen Reaktoren zur Alkandehydrierung sind außerdem zusätzliche Maßnahmen zur Verringerung von Stickoxiden im Abgas der Reaktoren bzw. ihrer Brennkammern erforderlich. Auch die mit der Verwendung einer Vielzahl von Brennern verbundene Geräuschentwicklung ist beträchtlich, weshalb in herkömmlichen Reaktoren aufwendige Lärmschutzmaßnahmen ergriffen werden müssen.

Die Erfindung stellt sich vor diesem Hintergrund die Aufgabe, herkömmliche Verfahren zur Alkandehydrierung, die mit den oben erläuterten Reaktoren arbeiten, zu verbessern.

### Offenbarung der Erfindung

Diese Aufgabe wird durch ein Verfahren und eine Vorrichtung zur Alkandehydrierung mit den Merkmalen der unabhängigen Patentansprüche gelöst. Bevorzugte Ausgestaltungen sind Gegenstand der abhängigen Patentansprüche und der nachfolgenden Beschreibung.

Die vorliegende Erfindung beruht unter anderem auf der Erkenntnis, dass sich der zuvor erwähnte ungleichmäßige Wärmeeintrag im Wesentlichen auf lokale Wärmespitzen in Form der Brennflammen der herkömmlicherweise eingesetzten Brenner zurückführen lässt. Die vorliegende Erfindung adressiert dieses Problem durch den Einsatz sogenannter flammenloser Brenner, also Brenner die zur flammenlosen Verbrennung bzw. Oxidation (engl. flameless combustion, flameless oxidation) eingerichtet sind. Die flammenlose Verbrennung ist grundsätzlich bereits seit längerem bekannt, ihre besonderen Vorteile für die Alkandehydrierung wurden jedoch nach diesseitiger Kenntnis bisher nicht erkannt. Aspekte der flammenlosen Verbrennung sind beispielsweise in der EP 0 463 218 A1 und der EP 0 685 683 A1 sowie einer Reihe einschlägiger Fachbücher beschrieben, beispielsweise bei Wünning, J.G. und Miano, A. "Handbuch der Brennertechnik für Industrieöfen", Vulkan-Verlag 2007, insbesondere Abschnitt 4.3, "Flammenlose Oxidation".

Eine flammenlose Verbrennung ergibt sich insbesondere dann, wenn ein Brenngas (unter einem "Brenngas" wird nachfolgend ein Reingas oder ein Gemisch mehrerer brennbarer Gase, beispielsweise Erdgas, verstanden) in einer Verbrennungszone eines Brenners mit einem Gasgemisch in Kontakt gebracht wird, das auf eine Temperatur aufgeheizt ist, die oberhalb der Zündtemperatur des Brenngases liegt. Bei dem entsprechend aufgeheizten Gasgemisch kann es sich um dem Brenner zugeführte Verbrennungsluft oder insbesondere auch um in die Verbrennungszone zurückgeführte Verbrennungsabgase handeln. Letztere können durch eine gezielt bewirkte interne Zirkulation in einer Brennkammer in die Verbrennungszone zurückgeführt werden. Die flammenlose Verbrennung setzt, wie anderenorts beschrieben, beispielsweise voraus, dass Brenngas und Verbrennungsluft vor der eigentlichen Verbrennungsreaktion in einen starken, umlaufenden Abgasstrom eingemischt werden. Eine Vorheizung der Verbrennungsluft selbst ist zwar keine Voraussetzung für die flammenlose Verbrennung, jedoch bietet sich diese zur Aufheizung und zur Ausbildung entsprechender zirkulierender Ströme an.

Bei der flammenlosen Verbrennung, bei der die Temperatur des Brennraums typischerweise bei 1000 °C oder mehr liegt, kann visuell keine Flamme mehr beobachtet werden und es ist kein entsprechendes UV-Signal mehr erkennbar. Dennoch wird auch in einem solchen Fall das Brenngas vollständig verbrannt.

### Vorteile der Erfindung

Die flammenlose Verbrennung hat den besonderen Vorteil, dass sie besonders vollständig erfolgt und der Kohlenmonoxidgehalt im Abgas damit auf sehr geringen Werten, typischerweise unterhalb von 1 ppm liegt. Gleichzeitig ergeben sich ausgesprochen geringe Stickoxidemissionen, die teilweise unterhalb der Nachweisgrenze liegen. Die Verbrennung erfolgt, wie im Rahmen der vorliegenden Erfindung von besonderem Vorteil, ausgesprochen stabil und gleichmäßig.

Die vorliegende Erfindung geht vor diesem Hintergrund von einem Verfahren zur Alkandehydrierung aus, bei dem ein Einsatzstrom, der ein oder mehrere zu dehydrierende Alkane enthält, in zumindest einen Reaktor durch eine Anzahl von Reaktionsrohren geführt wird, die in einer Brennkammer angeordnet sind und in der Brennkammer mit einer Anzahl von Brennern beheizt werden, denen ein Brenngas und Verbrennungsluft zugeführt wird.

Erfindungsgemäß ist, wie bereits angesprochen, vorgesehen, dass die Brenner zumindest teilweise flammenlos betrieben werden, indem einer Verbrennungszone dieser Brenner ein Gasgemisch zugeführt wird, das auf eine Temperatur oberhalb einer Zündtemperatur des Brenngases aufgeheizt ist.

Durch die erfindungsgemäß eingesetzten flammenlos betriebenen Brenner lassen sich insbesondere die zuvor angesprochenen Temperaturinhomogenitäten in Verfahren zur Alkandehydrierung nahezu vollständig aufheben. Die Wärmeverteilung und Übertragung von den Brennern zu den einzelnen Reaktionsrohren ist damit deutlich homogener im Vergleich zum Betrieb mit herkömmlich betriebenen Brennern. Insgesamt lässt sich nicht nur in der Longitudinalrichtung (d.h. in einer Richtung parallel zur Ausrichtung des jeweiligen Brenners) sondern auch zirkumferential eine nahezu einheitliche Wärmeausbreitung erzielen. Es ergibt sich insgesamt in der gesamten Brennkammer eine einheitliche Temperaturverteilung, d.h. es existieren kaum mehr Reaktionsrohre mit gegenüber einem Durchschnitt höherem bzw. niedrigerem Wärmeeintrag.

Weil durch den Einsatz flammenlos betriebener Brenner durch jeden einzelnen Brenner aufgrund der Vermeidung von Wärmespitzen eine jeweils höhere Leistung eingebracht werden kann, kann insgesamt eine geringere Anzahl von (jeweils mit höherer Leistung betriebenen) Brenner verwendet werden. Dies verringert die für die Brenner aufzuwendenden Kosten und den Aufwand zur Überwachung der Flammen und Pilotbrenner herkömmlich betriebener Brenner. Auch die eingangs erwähnte Geräuschentwicklung wird deutlich reduziert.

Durch die gleichmäßige Wärmeverteilung erhöht sich die Lebensdauer der Reaktionsrohre und des enthaltenen Katalysators, weil die Reaktionsrohre über ihre gesamte Länge und ihren gesamten Umfang unter Vermeidung von Temperaturspitzen gleichmäßig erwärmt werden.

Wie sich überraschend herausgestellt hat, sind die im Rahmen der vorliegenden Erfindung erzielbaren Produktausbeuten gegenüber der Verwendung herkömmlich betriebener Brenner deutlich erhöht. Als ursächlich hierfür werden zwei Effekte angenommen, nämlich die signifikante Verringerung der Ungleichverteilung des Wärmeeintrags zwischen den unterschiedlichen Reaktionsrohren einerseits und die Realisierung eines flachen, einheitlichen Wärmeflussprofils entlang der Länge der Reaktionsrohre, also in Longitudinalrichtung, andererseits. Details hierzu sind in den beigefügten Figuren veranschaulicht und dort nochmals erläutert.

Insgesamt ergibt sich auch eine verbesserte Katalysatorlebensdauer aufgrund der Vermeidung von Wärmespitzen, die zu einer überhöhten Katalysatortemperatur führen können. Entsprechende Reaktionsrohre können länger im Produktionsbetrieb belassen werden, weil die Gefahr lokaler Verkokungen durch die erläuterten Temperaturspitzen vermieden wird. Zusätzliche Maßnahmen zur Verringerung von Stickoxiden im Abgas des Reaktors bzw. seiner Brennkammer sind nicht mehr erforderlich, weil, wie erläutert, in flammenlos betriebenen Brennern die Stickoxidbildung deutlich geringer ist. Beim Einsatz flammenlos betriebener Brenner sind ferner auch geringere Anforderungen an die Exaktheit der Anordnung der Reaktionsrohre in der Brennkammer und/oder der Brenner zu beachten, da die Wärmeverteilung über die gesamte Brennkammer einheitlich ist.

Insgesamt ist in flammenlos betriebenen Brennern die Vermischungsgeschwindigkeit von Brenngas, Verbrennungsluft und Rauchgas wesentlich größer als die Kinetik der Verbrennung. Die schnelle Vermischung wird beispielsweise durch einen hohen Eintrittsimpuls (insbesondere der Verbrennungsluft) angetrieben und sorgt für ein gleichmäßiges Temperaturprofil im Feuerungsraum. Die Anzahl der Brenner wird nicht mehr durch die Rohranzahl und die Lage der Brenner zu den Rohren bestimmt sondern durch den Strömungsquerschnitt, damit insbesondere die Vermischung mit Rauchgas nicht behindert wird.

Als besonders vorteilhaft hat sich im Rahmen der vorliegenden Erfindung herausgestellt, als Brenngas für die verwendeten Brenner Erdgas oder Synthesegas einzusetzen und das Gasgemisch den Brennern auf einer Temperatur von mindestens 760 oder 850 °C und bis zu 900 oder 1000 °C zuzuführen. Wie erläutert, ist ein flammenloser Betrieb von Brennern nur oberhalb der Zündtemperatur des Brenngases möglich. Bei Erdgas liegt diese Temperatur bei ca. 760°C. Mit einem entsprechenden Sicherheitszuschlag im angegebenen Umfang, beispielsweise von 90°C, kann die erforderliche Temperatur sicher eingehalten werden. Als Brenngas kann auch ein aus einem Abstrom des Reaktors gewonnenes Restgas, das von zu gewinnenden Produkten abgetrennt wird, verwendet werden.

Unter einem Gasgemisch, das der Verbrennungszone zugeführt wird, sei im Rahmen dieser Erfindung insbesondere Verbrennungsluft, aber auch ein Rauchgas verstanden, das in die Verbrennungszone zurückgeführt wird, beispielsweise durch Einstellen entsprechender Strömungsbedingungen und/oder ein eine entsprechende Turbulenz erzeugendes Einspeisen von Verbrennungsluft, verstanden. Insbesondere setzt sich ein solches Gasgemisch aus Verbrennungsluft und Rauchgas zusammen. Die Verbrennungszone ist ein Bereich, in den mittels eines entsprechenden Brenners Brenngas in einer Brennkammer, ggf. zusammen mit Verbrennungsunterstützungsgas, eingebracht wird und in dem sich in einem herkömmlichen, nicht flammenlosen Betrieb eine Flamme ausbilden würde.

Es kann vorgesehen sein, die Verbrennungsluft, die von außen den Brennern zugeführt wird, auf eine entsprechende Temperatur vorzuheizen. Sind keine ausreichenden Wärmemengen verfügbar, um die Verbrennungsluft auf entsprechende Temperaturen vorzuheizen, beispielsweise weil ein Reaktor, dessen Abwärme zur Erwärmung der Verbrennungsluft verwendet wird, noch nicht auf Betriebstemperatur ist, schalten die verwendeten Brenner automatisch in den Flammenbetrieb.

Besonders vorteilhaft ist es, wenn die Verbrennungsluft mit Wärme vorgeheizt wird, die einem in den Reaktor aus dem Einsatzstrom gebildeten Produktstrom und/oder einem aus der Brennkammer entnommenen Abgasstrom in zumindest einem Wärmetauscher entzogen wird. Die Reaktionstemperaturen in entsprechenden Reaktoren liegen bei mindestens 1000 °C, beispielsweise bei 1050 bis 1100 °C, so dass entsprechende Abwärme in ausreichender Menge zur Verfügung steht und sinnvoll zur Aufheizung der Verbrennungsluft genutzt werden kann.

In einem entsprechenden Verfahren ist es insbesondere von Vorteil, wenn die Verbrennungsluft mit einem Überdruck von 10 bis 80 mbar, insbesondere von 20 bis 30 mbar oder von 55 bis 65 mbar (höhere Drücke sind bei der Verwendung der erläuterten Wärmetauscher erforderlich) bereitgestellt wird. Dies erlaubt die Einstellung der für die flammenlose Verbrennung erforderlichen Turbulenz in der Brennkammer.

Als besonders vorteilhaft hat sich ein Verfahren erwiesen, bei dem ein Reaktor mit einer Brennkammer verwendet wird, in der Reaktionsrohre zumindest in einem Abschnitt senkrecht verlaufen, wobei der Einsatzstrom von oben nach unten durch die Reaktionsrohre geführt wird und die Brenner in einem oberen Bereich der Brennkammer angeordnet sind. Der Gesamtstrom in einer entsprechenden Brennkammer verläuft auf diese Weise von oben nach unten, was eine intensive Verwirbelung mit den Verbrennungsabgasen ermöglicht.

Insbesondere können die Brenner dabei zumindest teilweise in einem Dach der Brennkammer und in Draufsicht zwischen den Reaktionsrohren verteilt angeordnet sein. Wie erläutert, sind hierbei geringere Anforderungen an die Exaktheit der Anordnung der Brenner und der Reaktionsrohre zu stellen, weil sich die Wärme in der Brennkammer aufgrund des flammenlosen Betriebs einheitlich verteilt. In einem derartigen Brenner sind vorteilhafterweise im Boden Rauchgasabzugsöffnungen angeordnet, da an dieser Stelle das Brenngas vollständig verbrannt ist.

Die Vollständigkeit der Verbrennung richtet sich in einem derartigen Brennraum insbesondere nach der Höhe des Brennraums, der vorteilhafterweise mindestens 5 m, insbesondere mindestens 8 m und bis zu 15 m, insbesondere bis zu 12 m, beträgt. Auf diese Weise kann sichergestellt werden, dass die Weglänge bis zum Rauchgasaustritt eine vollständige Verbrennung gewährleistet. Es kann davon ausgegangen werden, dass in entsprechenden Brennern nach 10 m Weglänge bis zu 99,9% des Brenngases verbrannt sind; dies kann jedoch auch bereits nach ca. 5 m Weglänge der Fall sein.

Alternativ zu der zuvor erläuterten Anordnung können die Brenner auch im Boden einer entsprechenden Brennkammer und die Rauchgasöffnungen in der Decke angeordnet sein. Es ist auch möglich, Brenner und Rauchgasöffnungen in einer Ebene, beispielsweise in einem oberen und einem unteren Bereich der Brennkammer, anzuordnen und durch zusätzliche Maßnahmen, beispielsweise eine entsprechende Rauchgasführung, sicherzustellen, dass durch die Rauchgasöffnungen nur vollständig verbrannter Brennstoff austritt. Auf diese Weise lässt sich die durch das Rauchgas zurückzulegende Wegstrecke verlängern.

Die vorliegende Erfindung eignet sich insbesondere für Verfahren zur Alkandehydrierung, bei denen ein Einsatzstrom verwendet wird, der Propan und/oder Butan enthält, wobei in den Reaktionsrohren ein Katalysator bereitgestellt ist, der eine Dehydrierung des Propans zu Propylen und/oder des Butans zu Butylen bewirkt. Wie eingangs erläutert und in der zitierten Fachliteratur beschrieben, richtet sich die Selektivität entsprechender Verfahren nach der Wahl des jeweils geeigneten Katalysators, die der Fachmann aufgrund von Angaben in der Fachliteratur vornimmt.

In entsprechenden Brennkammern können zumindest zwei Gruppen von Brennern verwendet werden, die mit einer unterschiedlichen Brennerleistung betrieben werden. Beispielsweise können randständige Brenner mit geringerer Brennerleistung betrieben werden, da diese nur einen kleineren Bereich aufheizen.

Eine Anlage zur Alkandehydrierung mit zumindest einem Reaktor mit einer Anzahl von Reaktionsrohren, die in einer Brennkammer angeordnet sind, und mit Mitteln, die dafür eingerichtet sind, einen Einsatzstrom, der ein oder mehrere zu dehydrierende Alkane enthält, durch die Reaktionsrohre zu führen und die Reaktionsrohre in der Brennkammer mit einer Anzahl von Brennern zu beheizen, denen ein Brenngas und Verbrennungsluft zugeführt wird, ist ebenfalls Gegenstand der vorliegenden Erfindung.

Erfindungsgemäß sind Mittel vorgesehen, die dazu eingerichtet sind, die Brenner zumindest teilweise flammenlos zu betreiben, indem einer Verbrennungszone dieser Brenner ein Gasgemisch zugeführt wird, das auf eine Temperatur oberhalb einer Zündtemperatur des Brenngases aufgeheizt ist.

Zu Merkmalen und Vorteilen einer entsprechenden Anlage, die insbesondere zur Durchführung eines zuvor erläuterten Verfahrens eingerichtet ist, sei auf die obigen Erläuterungen ausführlich verwiesen.

Wie bereits angesprochen, können in einer entsprechenden Brennkammer die Reaktionsrohre zumindest in dem Abschnitt der Brennkammer senkrecht verlaufen, wobei der Einsatzstrom von oben nach unten durch die Reaktionsrohre geführt wird und die Brenner in einem oberen Dach der Brennkammer und in Draufsicht zwischen den Reaktionsrohren verteilt angeordnet sind.

Die Erfindung wird nachfolgend unter Bezugnahme auf die beigefügten Figuren näher erläutert, die eine bevorzugte Ausführungsform der Erfindung gegenüber dem Stand der Technik zeigen.

### Kurze Beschreibung der Zeichnungen

Figur 1 zeigt eine Anlage gemäß dem Stand der Technik in vereinfachter, schematischer Darstellung in seitlicher Ansicht.
Figur 2 zeigt eine Anlage gemäß einer Ausführungsform der Erfindung in vereinfachter, schematischer Teildarstellung in Draufsicht.
Figur 3 veranschaulicht eine Wärmeverteilung in einer Anlage gemäß dem Stand der Technik in Form eines Diagramms.
Figur 4 veranschaulicht eine Wärmeverteilung in einer Anlage gemäß dem Stand der Technik und einer Ausführungsform der Erfindung in Form eines Diagramms.
Figur 5 veranschaulicht Kenngrößen von Verfahren gemäß dem Stand der Technik und einer Ausführungsform der Erfindung in Form von Diagrammen.

In den Figuren sind einander entsprechende Elemente mit identischen Bezugszeichen angegeben und der Übersichtlichkeit halber nicht wiederholt erläutert.

### Ausführliche Beschreibung der Zeichnungen

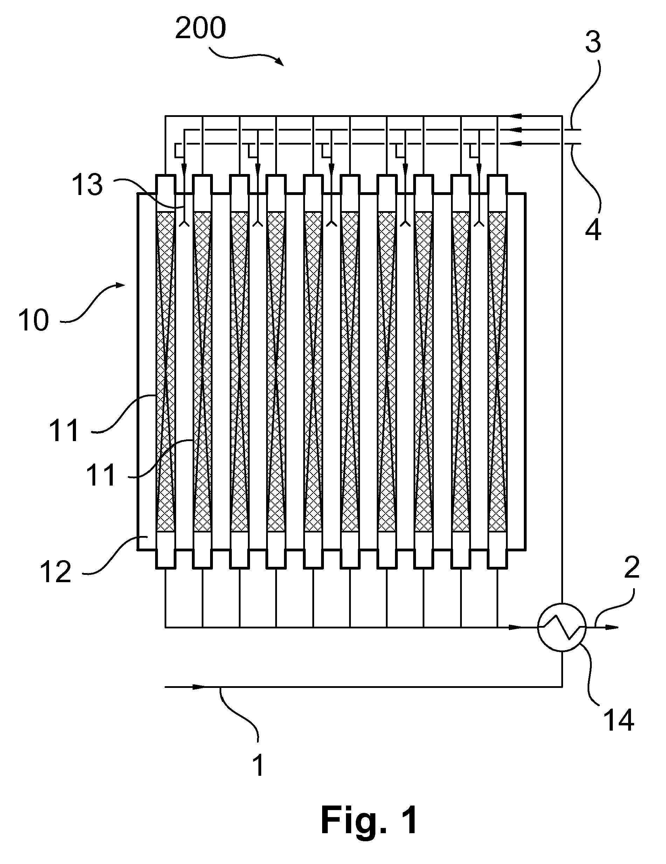

In Figur 1 ist eine Anlage zur Alkandehydrierung gemäß dem Stand der Technik in vereinfachter schematischer Darstellung in Seitenansicht gezeigt und insgesamt mit 200 bezeichnet.

Die Anlage 200 umfasst einen Reaktor 10 mit einer Anzahl von Reaktionsrohren 11 (nur teilweise beschriftet), die in einer Brennkammer 12 angeordnet sind. Ein Einsatzstrom 1, der ein oder mehrere zu dehydrierende Alkane enthält, wird in dem Reaktor 10 durch die Reaktionsrohre 11 geführt und zuvor mittels eines Wärmetauschers 14 mit einem in dem Reaktor 10 aus dem Einsatzstrom 1 gebildeten Produktstrom 2 vorgeheizt. In der Brennkammer 12 sind Brenner 13 angeordnet (ebenfalls nur teilweise beschriftet), die zwischen den Reaktionsrohren 11 verteilt in einem Dach der Brennkammer 12 angeordnet sind. Den Brennern wird jeweils Brenngas 3 und Verbrennungsluft 4 zugeführt.

Weitere Komponenten einer entsprechenden Anlage 200 sind nicht veranschaulicht, insbesondere ist die Erwärmung der Verbrennungsluft 4 nicht veranschaulicht. Auf eine Darstellung einer Produktaufbereitung, der der Produktstrom 2 unterworfen wird, wurde ebenfalls verzichtet. In den Reaktionsrohren 11 ist ein Katalysator bereitgestellt, der eine Alkandehydrierung, beispielsweise von in dem Einsatzstrom 1 enthaltenem Propan zu Propylen und/oder von Butan zu Buten, ermöglicht.

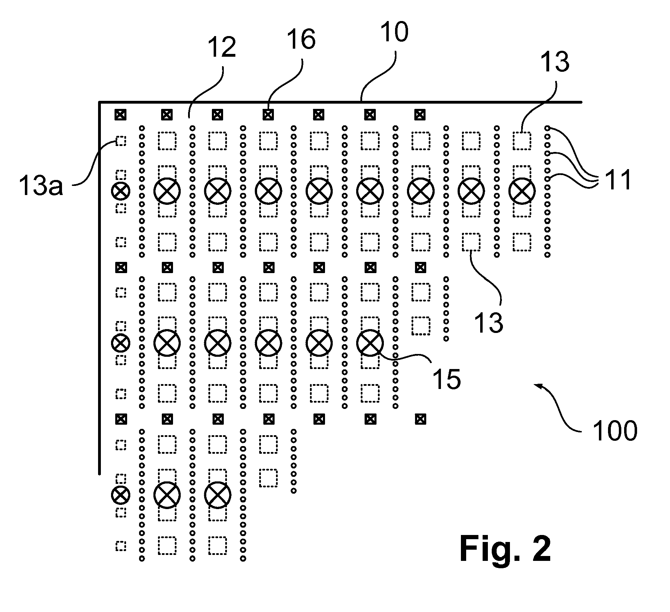

Figur 2 zeigt eine Anlage zur Alkandehydrierung gemäß einer Ausführungsform der Erfindung in vereinfachter schematischer Darstellung in Draufsicht, d.h. senkrecht zu einer Papierebene der Darstellung der Figur 1. Die Anlage ist insgesamt mit 100 bezeichnet. Gleiche Elemente wie in der Anlage 200 tragen wie erläutert, entsprechende Bezugszeichen. Die Figur 2 zeigt nur einen Teil eines Reaktors 10, die gezeigte Anordnung setzt sich in beliebiger Weise nach rechts und unten fort.

Wie ersichtlich, sind die Reaktionsrohre 11 (in der dargestellten Draufsicht als Kreise veranschaulicht und nur zum Teil beschriftet) des Reaktors 10 zu jeweils 16 Stück in mehreren nebeneinander angeordneten Reihen bereitgestellt. Ein entsprechender Reaktor 10 kann einige dutzend bis einige hundert Reaktionsrohre 11 umfassen.

Herkömmliche (Flammen-)Brenner, die in einer entsprechenden Anlage 100 bereitgestellt werden müssten, würde diese gemäß dem Stand der Technik ausgebildet, wie beispielsweise in einer Anlage 200 gemäß Figur 1, sind in Form gestrichelter Quadrate veranschaulicht und an einer Stelle exemplarisch mit 13 bezeichnet. Wie ersichtlich, können beispielsweise zwischen zwei Reihen von Reaktionsrohren 11 vier herkömmliche Brenner 13 angeordnet sein. Die Brenner am Rand der Brennkammer 12, in der Figur 2 an einer Stelle exemplarisch mit 13a bezeichnet, können beispielsweise kleiner dimensioniert und/oder mit geringerer Leistung betrieben werden.

Werden gemäß einer Ausführungsform der Erfindung flammenlos betriebene Brenner verwendet, die in der Figur 2 in Form diagonal durchkreuzter Kreise veranschaulicht und an einer Stelle exemplarisch mit 15 bezeichnet sind, können diese in sehr viel geringerer Zahl eingesetzt werden. Die Anzahl gegenüber den herkömmlichen Brennern 13 reduziert sich beispielsweise auf ein Viertel. Auf diese Weise kann eine für die flammenlose Verbrennung deutlich verbesserte Zirkulation von Verbrennungsabgas in der Brennkammer 12 erzielt werden. Auch hier können Brenner am Rand der Brennkammer 12 beispielsweise kleiner dimensioniert und/oder mit geringerer Leistung betrieben werden.

Die Brenner 15 sind zwischen den Reaktionsrohren 11 verteilt am Dach des Reaktors 10 angeordnet. Wie in der Figur 2 mit diagonal durchkreuzten Quadraten veranschaulicht und an einer Stelle exemplarisch mit 16 bezeichnet, können Rauchgasabzugsöffnungen vorgesehen sein, die am Boden einer entsprechenden Brennkammer 10 angeordnet sind.

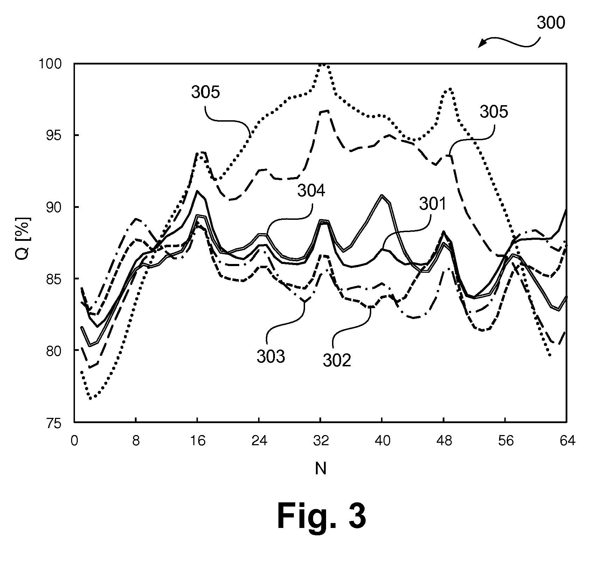

In Figur 3 ist eine Temperaturverteilung in einem Reaktor gemäß dem Stand der Technik in Form eines Diagramms 300 veranschaulicht.

Dem Diagramm 300 liegt ein Reaktor zugrunde, wie er grundsätzlich in Figur 2 veranschaulicht ist (allerdings ausschließlich mit herkömmlichen Flammenbrennern), und in dem eine beliebige Anzahl an Rohrreihen (entsprechend den senkrechten Rohrreihen der Figur 2) mit hier jeweils 64 Reaktionsrohren angeordnet sind. Ausgewählte Rohrreihen ist mit 301 bis 306 veranschaulicht, wobei die Rohrreihe 301 am Rand (vgl. ganz linke senkrechte Rohrreihe in Figur 2) und die Rohrreihen 302 bis 306 zunehmend mittig in einer Brennkammer des Reaktors angeordnet sind.

Die fortlaufende Nummer N (1 bis 64) eines Reaktionsrohrs in einer Rohrreihe ist auf der Abszisse des Diagramms 300 aufgetragen, wobei die kleinsten und größten fortlaufenden Nummern wiederum randständige und die mittleren fortlaufenden Nummern mittig angeordnete Reaktionsrohre angeben. Eine für die jeweiligen Reaktionsrohre in den Rohrreihen 301 bis 306 zur Verfügung stehende normierte Wärmemenge Q ist in Prozent auf der Ordinate aufgetragen.

Man erkennt, dass für die randständigen Rohrreihen, insbesondere die Rohrreihen 301 bis 303, und für die randständigen Reaktionsrohre einer jeden Rohrreihe 301 bis 306 ein deutlich geringeres Wärmeangebot zur Verfügung steht als für die mittig angeordneten Rohrreihen, beispielsweise die Rohrreihen 305 und 306 bzw. die mittig angeordneten Reaktionsrohre einer jeden Rohrreihe 301 bis 306, was zu den bereits eingangs erläuterten Problemen führen kann.

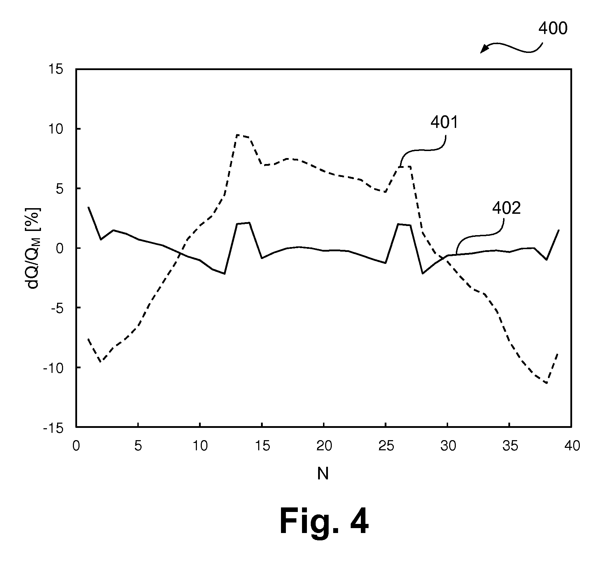

In Figur 4 ist eine Temperaturverteilung in einem Reaktor gemäß dem Stand der Technik und gemäß einer Ausführungsform der Erfindung in Form eines Diagramms 400 veranschaulicht.

Dem Diagramm 400 liegt ein Reaktor zugrunde, in dem eine Rohrreihe mit 39 Reaktionsrohren angeordnet ist.

Die fortlaufende Nummer N (1 bis 39) eines Reaktionsrohrs in der Rohrreihe ist auf der Abszisse des Diagramms 400 aufgetragen, wobei die kleinsten und größten fortlaufenden Nummern erneut randständige und die mittleren fortlaufenden Nummern mittig angeordnete Reaktionsrohre angeben. Eine für das jeweilige Reaktionsrohr in der Rohrreihe als Abweichung dQ zu einem Mittelwert Q_{M} für alle Reaktionsrohre zur Verfügung stehende Wärmemenge dQ/Q_{M} ist in Prozent auf der Ordinate aufgetragen.

Mit Reihe 401 ist eine Wärmeverteilung veranschaulicht, wie sie sich in einem mit herkömmlichen Brennern 13 betriebenen Reaktor 10 bzw. dessen Brennkammer 12 ergibt, mit Reihe 402 eine Wärmeverteilung bei Einsatz von flammenlos betriebenen Brennern 15 gemäß einer Ausführungsform der Erfindung. Wie ersichtlich, ist die Ungleichverteilung in der Reihe 402 deutlich reduziert.

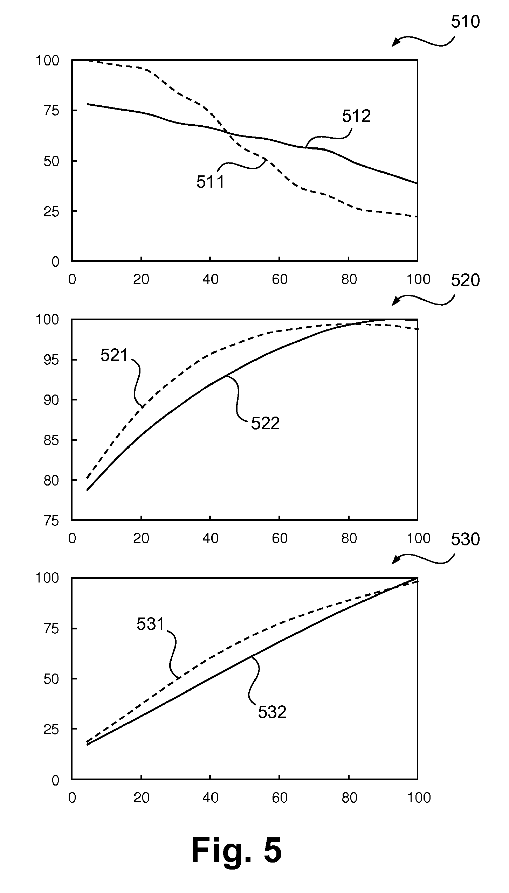

In der Figur 5 sind Kenngrößen herkömmlich und erfindungsgemäß beheizter Reaktionsrohre in Form entsprechender Diagramme 510, 520 und 530 dargestellt. Den Diagrammen 510 bis 530 liegt jeweils ein Reaktionsrohr mit einer auf 100 normierten Länge zugrunde, das mit einem propanreichen Einsatzstrom beschickt wird.

In dem Diagramm 510 ist dabei eine auf 100 normierte absorbierte Wärme in dem Reaktionsrohr auf der Ordinate gegenüber der normierten Rohrlänge auf der Abszisse aufgetragen. Mit 511 ist ein Verlauf für eine Beheizung mit einem konventionell betriebenen Brenner 13, mit 512 ein Verlauf für eine Beheizung mit einem flammenlos betriebenen Brenner 15 dargestellt. Wie ersichtlich, ergibt sich insgesamt zwar eine geringere absorbierte Wärmemenge bei der Verwendung flammenlos betriebener Brenner 15, diese ist jedoch deutlich homogener. Wie ebenfalls ersichtlich wird bei der Verwendung konventioneller Brenner 13 am Beginn eines entsprechenden Reaktionsrohres eine vergleichsweise große Wärmemenge absorbiert, die sich jedoch später deutlich verringert. Hierdurch kommt es zu einer deutlichen Ungleichverteilung.

In dem Diagramm 520 ist eine auf 100 normierte Gastemperatur in einem entsprechenden Reaktionsrohr auf der Ordinate gegenüber der normierten Rohrlänge auf der Abszisse aufgetragen. Ein sich für eine Beheizung mit einem konventionell betriebenen Brenner 13 ergebender Verlauf ist mit 521, ein sich für eine Beheizung mit einem flammenlos betriebenen Brenner 15 ergebender Verlauf mit 522 angegeben. Bei einer Beheizung mit einem flammenlos betriebenen Brenner 15 ergibt sich ein deutlich flacherer Temperaturanstieg ohne Temperaturspitzen und abrupte Gradienten.

Wie aus dem Diagramm 530 ersichtlich, in welchem eine auf 100 normierte Propylenkonzentration auf der Ordinate gegenüber der normierten Rohrlänge auf der Abszisse aufgetragen ist, ergibt sich hier zwischen einer Beheizung mit einem konventionell betriebenen Brenner 13, wie mit Verlauf 531 veranschaulicht, und einer Beheizung mit einem flammenlos betriebenen Brenner 15, wie mit Verlauf 532 veranschaulicht, ebenfalls ein Unterschied. Insgesamt führt der flammenlose Betrieb entsprechender Brenner zu deutlich höheren Propylenkonzentrationen, die Propylenkonzentration steigt ferner im Verlauf des Reaktionsrohrs bzw. über die Strecke eines entsprechenden Reaktionsrohrs homogener an.

## Patentansprüche

1. Verfahren zur Alkandehydrierung, bei dem ein Einsatzstrom (1), der ein oder mehrere zu dehydrierende Alkane enthält, in zumindest einem Reaktor (10) durch eine Anzahl von Reaktionsrohren (11) geführt wird, die in einer Brennkammer (12) angeordnet sind und in dieser mit einer Anzahl von Brennern (15) beheizt werden, denen zumindest ein Brenngas (3) zugeführt wird, **dadurch gekennzeichnet, dass** die Brenner (15) zumindest zeitweise flammenlos betrieben werden, indem einer Verbrennungszone der Brenner (15) ein Gasgemisch auf einer Temperatur, die oberhalb einer Zündtemperatur des Brenngases (3) liegt, zugeführt wird.

2. Verfahren nach Anspruch 1, bei dem als Brenngas Erdgas und/oder Synthesegas und/oder ein aus einem Abstrom des Reaktors (10) gewonnenes Restgas verwendet wird und bei dem das Gasgemisch den Brennern (15) auf einer Temperatur von mindestens 750 bis 1000 °C zugeführt wird.

3. Verfahren nach Anspruch 1 oder 2, bei dem das Gasgemisch rückgeführte Verbrennungsabgase der Brenner (15) und Verbrennungsluft (4) umfasst.

4. Verfahren nach Anspruch 3, bei dem die Verbrennungsluft (4) mit Abwärme vorgeheizt wird, die einem in dem Reaktor (10) gebildeten Produktstrom (2) und/oder einem aus der Brennkammer (12) abgezogenen Abgasstrom in zumindest einem Wärmetauscher (14) entzogen wird.

5. Verfahren nach Anspruch 3 oder 4, bei dem die Verbrennungsluft (4) mit einem Überdruck von 10 bis 80 mbar, insbesondere von 20 bis 30 mbar oder von 55 bis 65 mbar, bereitgestellt wird.

6. Verfahren nach einem der vorstehenden Ansprüche, bei dem ein Reaktor (10) mit einer Brennkammer (12) verwendet wird, in der die Reaktionsrohre (11) zumindest in einem Abschnitt senkrecht verlaufen, wobei der Einsatzstrom (1) von oben nach unten durch die Reaktionsrohre (11) geführt wird und die Brenner (15) in einem oberen Bereich der Brennkammer (10) angeordnet sind.

7. Verfahren nach Anspruch 6, bei dem die Brenner (15) zumindest teilweise in einem Dach der Brennkammer (12) und in Draufsicht zwischen den Reaktionsrohren (11) verteilt angeordnet sind.

8. Verfahren nach Anspruch 7, bei dem die Brennkammer (12) in ihrem Boden Rauchgasabzugsöffnungen aufweist.

9. Verfahren nach einem der Ansprüche 6 bis 8, bei dem die Brennkammer (12) eine Höhe von mindestens 5 m, insbesondere mindestens 8 m, und bis zu 15 m, insbesondere bis zu 12 m, aufweist.

10. Verfahren nach einem der vorstehenden Ansprüche, bei dem ein Einsatzstrom (1) verwendet wird, der Propan und/oder Butan enthält, und wobei in den Reaktionsrohren (11) ein Katalysator bereitgestellt ist, der eine Dehydrierung des Propans zu Propylen und/oder des Butans zu Butylen bewirkt.

11. Verfahren nach einem der vorstehenden Ansprüche, bei dem zumindest zwei Gruppen von Brennern (15) verwendet werden, die mit einer unterschiedlichen Brennerleistung betrieben werden.

12. Anlage zur Alkandehydrierung mit zumindest einem Reaktor (10) mit einer Anzahl von Reaktionsrohren (11), die in einer Brennkammer (12) angeordnet sind, und mit Mitteln, die dafür eingerichtet sind, einen Einsatzstrom (1), der ein oder mehrere zu dehydrierende Alkane enthält, durch die Reaktionsrohre (11) zu führen und die Reaktionsrohre (11) mit einer Anzahl von Brennern (15) zu beheizen, denen zumindest ein Brenngas (3) zugeführt wird, **dadurch gekennzeichnet, dass** Mittel vorgesehen sind, die eingerichtet sind, einer Verbrennungszone der Brenner (15) ein Gasgemisch auf einer Temperatur, die oberhalb einer Zündtemperatur des Brenngases (3) liegt, zuzuführen und die Brenner (15) hierdurch zumindest zeitweise flammenlos zu betreiben.

13. Anlage nach Anspruch 12, die zur Durchführung eines Verfahrens nach einem der Ansprüche 1 bis 11 eingerichtet ist.

14. Anlage nach Anspruch 12 oder 13, bei der die Reaktionsrohre (11) zumindest in einem Abschnitt der Brennkammer (12) senkrecht verlaufen, wobei Mittel vorgesehen sind, die dafür eingerichtet sind, den Einsatzstrom (1) von oben nach unten durch die Reaktionsrohre (11) zu führen, und wobei die Brenner (15) in einem oberen Dach der Brennkammer (10) und in Draufsicht zwischen den Reaktionsrohren (11) verteilt angeordnet sind.
